# EUROPÄISCHE PATENTSCHRIFT

(11) **EP 2 155 316 B1**
(45) Veröffentlichungstag und Bekanntmachung des Hinweises auf die Patenterteilung: **02.02.2011**
(21) Anmeldenummer: 07856341.8
(22) Anmeldetag: 03.12.2007
(51) Int. Cl.: A61M 37/00, A61M 5/315

(54) **SPRITZENVORRICHTUNG MIT EINEM LÄNGLICHEN KOLBENRAUM**
SYRINGE DEVICE WITH AN ELONGATE PLUNGER SPACE
DISPOSITIF D'INJECTION AVEC ESPACE DE PISTON ALLONGÉ

(30) Priorität: 07.05.2007 DE 102007021243
(43) Veröffentlichungstag der Anmeldung: 24.02.2010
(73) Patentinhaber: Rexam Pharma GmbH, 79395 Neuenburg (DE)
(72) Erfinder: BIRMELIN, Uwe, 79379 Hügelheim (DE); EBERL-LANG, Martin, 81543 München (DE)
(74) Vertreter: Maucher, Wolfgang
(86) Internationale Anmeldenummer: PCT/EP2007/010492
(87) Internationale Veröffentlichungsnummer: WO 2008/135074

(56) Entgegenhaltungen:
- EP-A- 0 292 936
- EP-A- 0 564 038
- EP-B- 1 323 450
- DE-C1- 19 734 385
- US-A- 5 370 628

## Beschreibung

Die Erfindung betrifft eine Spritzenvorrichtung mit einem länglichen Kolbenraum oder Zylinder, mit einer Aufnahmekammer zum Aufnehmen einer oder mehrerer Feststoffmedikamente, mit einer an dem Kolbenraum angeordneten Hohlnadel und mit einem zu einer Bewegung in dem Kolbenraum oder Zylinder geeigneten Kolben, der einen Stößel zum Ausschieben des Feststoffmedikaments durch und aus der Hohlnadel aufweist, wobei eine an dem Kolben angreifende Kolbenstange für ihre Benutzung teleskopartig verlängerbar beziehungsweise relativ zu dem Kolben entgegen dessen Gebrauchsbewegung ausziehbar und mittels einer Verriegelung mit dem Kolben kuppelbar ist.

Eine derartige Spritzenvorrichtung ist aus EP 1 323 450 B1 bekannt und hat sich bewährt. Die gesamte Spritzenvorrichtung ist zunächst sehr kompakt und ihre wichtigen Teile sind weitgehend innerhalb dem gehäuseartigen Kolbenraum, von diesem geschützt und gegen Beschädigungen gesichert, untergebracht. Wird die teleskopartig zurückziehbare Kolbenstange aus diesem Kolbenraum zurückbewegt, wird sie relativ zu dem Kolben soweit bewegt, bis sie ihrerseits in Verriegelungs- oder Kupplungsposition mit dem Kolben gelangt, so dass danach ein Einschieben der Kolbenstange auch den Kolben verschiebt und dadurch das zunächst in einer Klemmhülse gehaltene Feststoffmedikament aus dieser und aus der Aufnahmekammer in und durch die Hohlnadel bewegt und schließlich appliziert wird.

Die Handhabung dieser Spritzenvorrichtung hat ergeben, dass bei deren Benutzung unter Umständen versäumt werden kann, die teleskopierbar zurückziehbare Kolbenstange tatsächlich soweit zurückzuziehen, dass sie in Verriegelungsstellung mit dem Kolben gelangt, so dass bei einem anschließenden Einschieben der Kolbenstange keine oder keine vollständige Applikation des Medikaments erfolgt.

Es besteht deshalb die Aufgabe, eine Spritzenvorrichtung der eingangs definierten Art zu schaffen, mit welcher Fehlbedienungen oder Fehlapplikationen weitestgehend verhindert werden können.

Zur Lösung dieser Aufgabe ist die eingangs definierte Spritzenvorrichtung dadurch gekennzeichnet, dass die teleskopartig relativ zueinander verstellbaren Teile oder Bereiche des Kolben und der Kolbenstange wenigstens eine oder mehrere gegenseitige Sperren für Zwischenpositionen der relativ zu dem Kolben ausziehbaren Kolbenstange aufweisen, welche Sperren bei nur teilweise relativ zu dem Kolben zurückgezogener Kolbenstange eine Verschiebung in Applikationsrichtung verhindern, und dass eine Kupplung oder Verriegelung des in seiner Ausgangslage befindlichen Kolbens in seiner Ausgangslage vorgesehen ist, die durch die in Gebrauchsstellung zurückgezogene Kolbenstange, in welcher die Kolbenstange mit dem Kolben in Schieberichtung verbunden ist, lösbar ist.

Wird bei einer derartigen Vorrichtung die Kolbenstange nicht weit genug zurückgezogen, um die erforderliche Gebrauchsstellung an dem Kolben zu erreichen, kann sie aufgrund der Sperren nicht in Applikationsrichtung verschoben werden, so dass der Benutzer den Kolben nur dann in Gebrauchsrichtung verschieben kann, wenn er die Kolbenstange weiter zurückzieht, bis sie tatsächlich ihre Endlage und damit die Kupplungsposition mit dem Kolben erreicht hat und dadurch der Kolben gegenüber dem Kolbenraum oder Gehäuse entriegelt ist. Da eine Verschiebung der Kolbenstange in Applikationsrichtung bei unzureichender Zurückziehung gesperrt ist, merkt der Benutzer, dass er die Gebrauchsstellung noch nicht erreicht hat, so dass vermieden werden kann, dass keine oder nur eine unzureichende Applikation durch eine Verschiebung der Kolbenstange aus einer Zwischenposition erfolgt. Die Sperrung der Kolbenstange in Zwischenstellungen gegen eine Einschubbewegung erfolgt also über die Einschnappverbindung an dem Kolben, der seinerseits mit dem Kolbenraum formschlüssig gekuppelt ist, so dass erst nach dem vollständigen Zurückziehen der Kolbenstange und dem dadurch gleichzeitig bewirkten Entriegeln des Kolbens in erwünschter Weise die Applikation erfolgen kann.

An dem Kolben kann ein nachgiebiger oder in den Umriss des Kolbens verstellbarer Vorsprung vorgesehen sein, der bei in Ausgangslage zurückgezogenem Kolben an einer Kupplungsstelle des Kolbenraums selbsttätig in eine Kupplungsvertiefung eingreift. Dadurch wird verhindert, dass der Kolben aus seiner Ausgangslage verschoben werden kann, bevor diese Kupplung durch die zurückgezogene Kolbenstange gelöst ist.

Der an dem Kolben angeordnete, selbsttätig in Kupplungsposition verstellbare Vorsprung kann eine Rast- oder Sperrklinke sein, die in Kupplungsstellung an einer sie untergreifenden Stelle des Kolbenraums oder seines Gehäuses eingreift. Dadurch befindet sich diese Klinke in der Kupplungsposition in dem Weg der teleskopartig zurückziehbaren Kolbenstange, so dass sie durch diese beaufschlagt und in Löseposition verstellt werden kann.

Günstig ist es dabei, wenn die an dem Kolben angeordnete Sperrklinke durch eine mit der Sperrklinke verbundene Führungsfläche und einen an der Kolbenstange angeordneten Vorsprungsbereich aus ihrer Kupplungs- oder Sperrstellung in den Umriss des Kolbens gegen ihre Rückstellkraft verstellbar ist, welcher Vorsprungbereich an der Kolbenstange nahe deren der Hohlnadel zugewandten Ende angeordnet ist. Dies ergibt eine zweckmäßige geometrische Zuordnung der Sperrklinke und ihrer Führungsfläche zu einem Vorsprungbereich an der Kolbenstange, wodurch die Entriegelung beziehungsweise Lösung der Kupplung zwischen Kolben und Kolbenraum etwa gleichzeitig mit dem Verriegeln der Kolbenstange mit dem Kolben erfolgen kann.

Günstig ist es, wenn der Kolben innerhalb der ein Hohlprofil bildendes oder rinnenförmiges Querschnittsprofil aufweisenden Kolbenstange angeordnet und diese den Kolben und wenigstens einen Teil seines Stößels vor der Gebrauchsstellung in sich aufnimmt. Dies ergibt die angestrebte platzsparende Anordnung, bei welcher die Länge des Stößels und die der Kolbenstange einander etwa entsprechen, aber durch das Ineinanderliegen nicht zu einer entsprechend langen Anordnung führen.

Die relativ zueinander in Längsrichtung teleskopartig verstellbaren Teile, nämlich die Kolbenstange und der relativ zu ihr verschiebbare Kolben - zu dem in Gebrauchsstellung eigentlich die Kolbenstange verschiebbar ist - können als gegenseitige Sperren in mehreren Zwischenstellungen Schnappverbindungen aufweisen, die eine Kupplung zwischen Kolbenstange und Kolben in Betätigungsrichtung des Kolben bewirken, welche Betätigung des Kolbens aber durch die Kupplung des Kolbens mit dem Kolbenraum mittels Sperrklinke in den Zwischenstellungen gesperrt ist oder bleibt. Bei der Verschiebung der Kolbenstange durch Zurückziehen aus dem Kolbenraum heraus relativ zu dem gesperrten Kolben ergeben sich also insbesondere mehrmals Positionen in denen die Kolbenstange mit dem Kolben in eine in axialer Richtung wirksame formschlüssige Verbindung gelangt, die aber nicht geeignet ist den Kolben zu verschieben, weil dieser bei diesen Verriegelungen in Zwischenposition seinerseits an dem Kolbenraum gekuppelt und damit verriegelt und gesperrt bleibt. Der Benutzer bemerkt aber in den Fällen, in denen er bei nur teilweiser Zurückziehung der Kolbenstange eine Applikation versucht, dass diese aufgrund der beschriebenen Sperrung nicht möglich ist, so dass er gezwungen ist, die Kolbenstange weiter zurückzuziehen, bis sie ihre Gebrauchsstellung erreicht hat und dadurch der Kolben für die Gebrauchsbewegung entriegelt wird.

Die Schnappverbindungen können durch an der Innenseite der Kolbenstange angeordnete Rampen oder Zungen gebildet sein, die ihrerseits mit einer insbesondere federelastischen Zunge an dem Kolben zusammenwirken, welche Zunge durch die Rampen beim Zurückziehen der Kolbenstange zunächst eindrückbar ist und jeweils hinter der Rampe durch ihre Rückstellkraft insbesondere hörbar in ihre Sperrrichtung bewegbar ist. Solche durch Rampen und darüber gleitende federelastische Zungen gebildete Gesperre sind vielfältig bekannt und stellen eine einfache Möglichkeit dar, eine in einer Richtung mögliche Bewegung in der Gegenrichtung zu sperren. Wenn der Vorgang dieses Einschnappens oder Sperrens hörbar ist, beispielsweise durch ein Klickgeräusch, wird auch der Benutzer besser auf diesen jeweiligen Einschnappvorgang aufmerksam, weil er auch akustisch darauf hingewiesen werden kann.

Besonders günstig ist es, wenn - wie eigentlich aus EP 1 323 450 B1 auch schon bekannt - die Kolbenstange als rinnenartiges Teil ausgebildet ist, in dessen Innenquerschnitt der Kolben über einen Teil seines Querschnitts umfasst und geführt ist, und wenn der Kolben die freien Ränder des U-Querschnitts der Kolbenstange mittels Vorsprüngen seitlich übergreift, die zusammen mit der Kolbenstange in den Kolbenraum passen und dessen Innenquerschnitt ausfüllen, also von dessen Innenquerschnitt geführt sind. Somit überragt der Kolben die U-förmig ausgebildete Querschnittsform der Kolbenstange und ergänzt diese zu einem zweckmäßigerweise rechteckigen Querschnitt, der in den entsprechenden rechteckigen Innenquerschnitt des Kolbenraums passt, wobei die Überstände des Kolbens gegenüber der Kolbenstange gleichzeitig für dessen Verriegelung an dem Kolbenraum ausgenutzt werden können.

Dabei ist es durch diese Geometrie zwischen Kolben und Kolbenstange möglich, dass die die Sperrklinke aufweisende Seite des Kolbens zum Abtrennen der Sperrklinke von der Kolbenseitenfläche geschlitzt ist und die außerhalb der Kolbenstange befindliche Begrenzung dieses Schlitzes über den Kolben seitlich etwa um die Dicke der Seitenwand der Kolbenstange übersteht, so dass der Kolben an seinem außerhalb des Querschnittes der teleskopierbaren Kolbenstange befindlichen Bereich eine Gesamtbreite hat, die der Gesamtbreite der Kolbenstange und der inneren Breite des Kolbenraumes, der praktisch die Spritzenkammer ist, entspricht.

Insgesamt ergibt sich eine Spritzenvorrichtung, bei welcher die Vorteile der Spritzenvorrichtung gemäß EP 1 323 450 B1 erhalten bleiben, insbesondere was die platzsparende Anordnung vor der Benutzung und die gute Halterung des Feststoffmedikaments in einer Klemmhülse innerhalb der Aufnahmekammer betrifft, wobei aber gleichzeitig erreicht wird, dass der Benutzer gezwungen ist, die Kolbenstange soweit zurückzuziehen, dass die Kupplung zwischen Kolben und Kolbenkammer gelöst wird und erst dann die Applikation des Medikaments möglich ist.

Nachstehend ist ein Ausführungsbeispiel der Erfindung anhand der Zeichnung näher beschrieben. Es zeigt in zum Teil schematisierter Darstellung:
- Fig. 1: eine Draufsicht
- Fig. 2: einen Längsschnitt gemäß der Linie II-II in Fig. 1 einer erfindungsgemäßen Spritzenvorrichtung in Aus- gangsstellung vor dem Zurückziehen der Kolbenstange und dem Entriegeln des Kolbens,
- Fig. 3: eine der Fig. 2 entsprechende Schnittdarstellung, allerdings in um 180° um die Längsachse gewendete Po- sition nach dem Abnehmen einer Schutzkappe und nach dem teleskopartigen Herausziehen der Kolbenstange aus dem der Hohlnadel abgewandten Ende des Kolbenraumes zum Verriegeln mit dem Kolben,
- Fig. 4: eine der Fig. 1 entsprechende entgegengesetzte An- sicht der Spritzenvorrichtung nach dem Zurückziehen der Kolbenstange in ihre Gebrauchsstellung und dem damit einhergehenden Kuppeln mit dem Kolben und Ent- riegeln dieses Kolbens gegenüber dem Kolbenraum,
- Fig. 5: eine der Fig. 3 entsprechende Darstellung nach dem Vorschieben der Kolbenstange und damit des Kolbens in Applikationsrichtung, wodurch ein Feststoffmedikament aus der Kanüle ausgeschoben ist gemäß der Schnitt- linie V-V in Fig. 6,
- Fig. 6: eine der Fig. 4 entsprechende Ansicht nach dem Ein- schieben der Kolbenstange in den Kolbenraum und damit dem Vorschieben des Kolbens sowie dem Ausschieben des Feststoffmedikaments,
- Fig. 7: eine Draufsicht nur der Kolbenstange und des Kolbens in Ausgangsstellung gemäß Fig. 1 und 2 mit Blick auf die offene Seite der im Querschnitt rinnenförmigen oder U-förmigen Kolbenstange in der Ausgangslage ge- mäß den Fig. 1 und 2,
- Fig. 8: in schaubildlicher Darstellung die Anordnung nur des Kolbens, seines Stößels und der Kolbenstange nach dem Zurückziehen der Kolbenstange in die Lage gemäß den Fig. 3 und 4 ohne den diese Anordnung in Gebrauchs- stellung aufnehmenden Kolbenraum,
- Fig. 9: eine schaubildliche Ansicht aus etwas anderem Blick- winkel der Anordnung gemäß Fig. 8,
- Fig. 10: eine Seitenansicht der Anordnung nach Fig. 8 mit Blick auf den einen U-Schenkel der im Querschnitt U- förmigen Kolbenstange nach deren Zurückziehen relativ zu dem Kolben und dem Beaufschlagen der Sperrklinke des Kolbens, womit dieser vor dem Zurückziehen der Kolbenstange mit dem in Fig. 7 bis 10 nicht darge- stellten Kolbengehäuse gekuppelt war,
- Fig. 11: einen Teillängsschnitt des Kolbenraums und des Kol- bens in Verriegelungsposition mit Blick auf die gemäß Fig. 4 zurückgezogene Kolbenstange, wobei eine lös- bare Sperrklinke am Kolben in Sperrstellung im Zu- sammenwirken mit dem Kolbenraum erkennbar ist,
- Fig. 12: in schaubildlicher Darstellung die Verriegelung des Kolbens gegenüber dem Kolbenraum bei zurückgezogener Kolbenstange, wobei Kolben und Kolbenstange sich in der in Fig. 8 dargestellten Lage befinden,
- Fig. 13: eine schaubildliche Darstellung der Verriegelung des Kolbens gegenüber dem Kolbenraum bei teilweise oder ganz zurückgezogener Kolbenstange unter einem gegen- über Fig. 12 etwa um 90° versetzten Blickwinkel sowie
- Fig. 14: eine Seitenansicht der erfindungsgemäßen Spritzenvor- richtung analog der Schnittdarstellung von Fig. 3 mit Blick auf die Verriegelung des Kolbens mit dem Kol- benraum.

Eine im Ganzen mit 1 bezeichnete Spritzenvorrichtung weist im Großen und Ganzen einen Aufbau etwa analog der Vorrichtung auf, die in EP 1 323 450 B1 beschrieben ist und auch in der Praxis entsprechend angewendet wird.

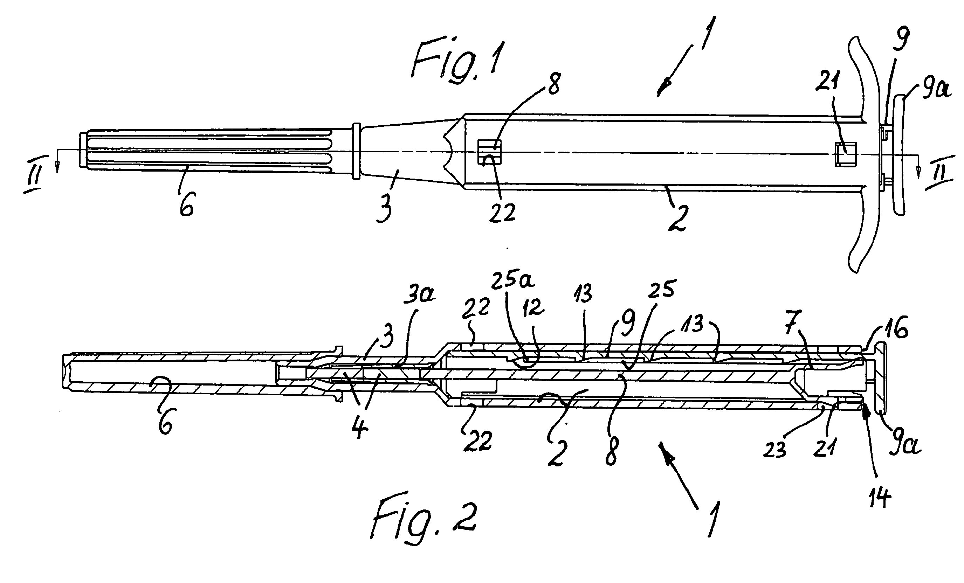

In der Zeichnung erkennt man, dass die Spritzenvorrichtung 1 einen länglichen, gehäuseartigen Kolbenraum 2 aufweist, der auch als Zylinder gestaltet sein könnte, im Ausführungsbeispiel aber einen rechteckigen Querschnitt hat, wie sich vor allem aus der gemeinsamen Betrachtung aller Figuren ergibt. An den Kolbenraum 2 ist eine Aufnahmekammer 3 angeschlossen, die zum Aufnehmen einer Klemmhülse 3a für ein oder mehrere darin gehaltene Feststoffmedikamente 4 dient, wie man es in den Fig. 2, 3, 5 und 6 erkennt.

An dem Kolbenraum 2 ist außerdem in Verlängerung der Aufnahmekammer 3 zumindest bei der Benutzung der Spritzenvorrichtung 1 eine Hohlnadel 5 angeordnet und angeschlossen, nach deren Einstechen in eine Applikationsstelle das Feststoffmedikament 4 appliziert werden kann.

In den Fig. 1 und 2 ist eine Schutzkappe 6 erkennbar, die die Hohlnadel 5 überdecken kann oder die zunächst entfernt werden muss, um die Hohlnadel 5 in Gebrauchsstellung bringen zu können.

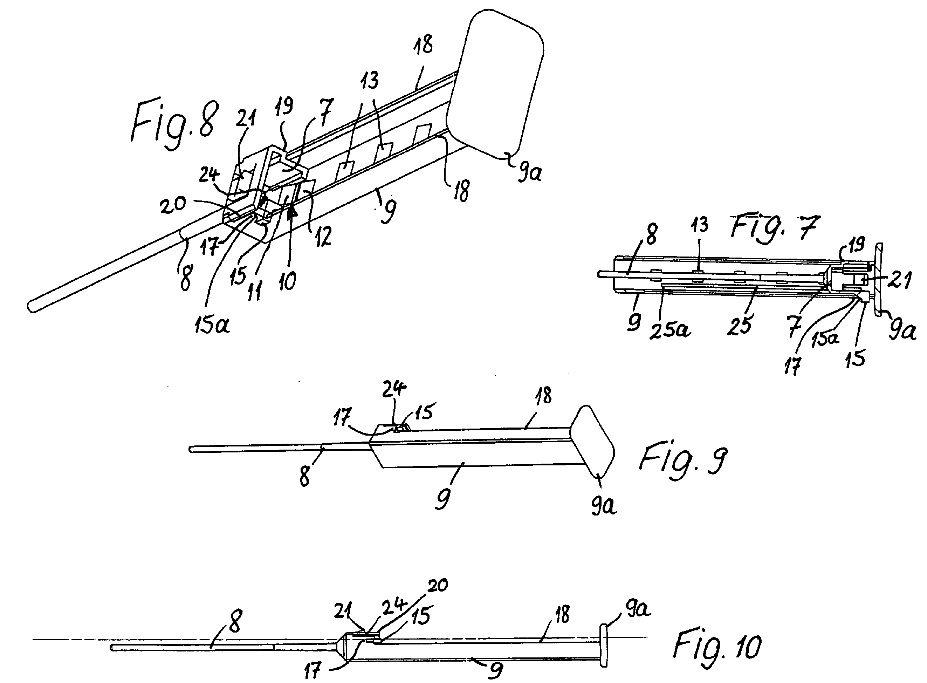

Für das Ausschieben des oder der Feststoffmedikamente 4 aus der Aufnahmekammer 3 und durch die Hohlnadel 5 hindurch ist in dem Kolbenraum 2 ein Kolben 7 vorgesehen, der einen Stößel 8 zum Beaufschlagen und Ausschieben des Feststoffmedikaments 4 durch die Hohlnadel 5 und aus dieser heraus trägt, wobei dieser Stößel 8 in Längserstreckungsrichtung der Spritzenvorrichtung 1 und in Bewegungsrichtung des Kolbens vor diesem angeordnet ist, wie man es deutlich in den Fig. 2, 3 und 5 sowie auch in den Fig. 7 bis 10 erkennt.

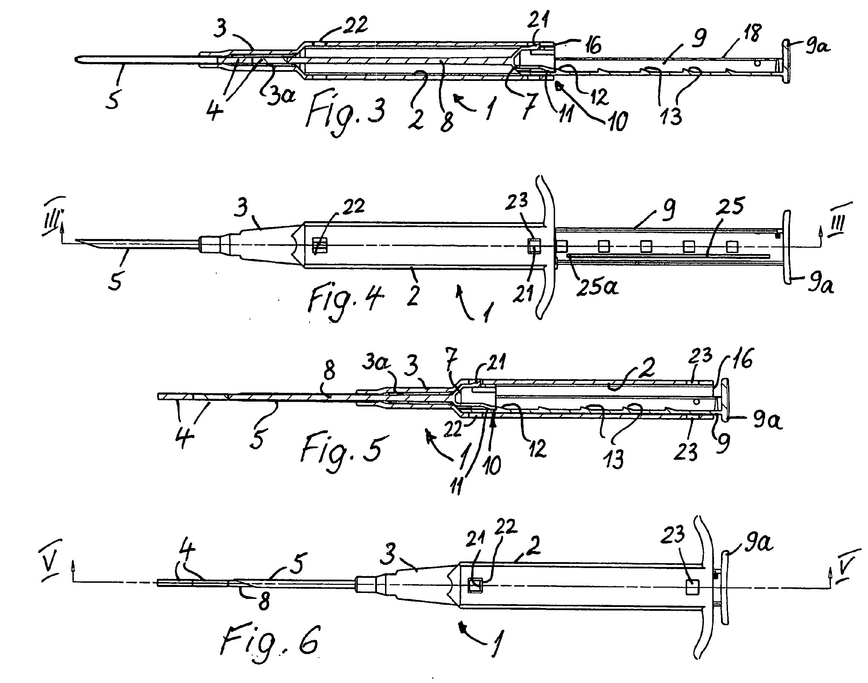

Für diese Bewegung des Kolbens 7 aus seiner in den Fig. 2 und 3 erkennbaren Ausgangslage in die Position gemäß Fig. 5 bis 8, 9 und 10, welche Kolbenbewegung das erwähnte Ausschieben des Feststoffmedikaments 4 bewirkt, ist eine an dem Kolben 7 angreifende, im Ganzen mit 9 bezeichnete Kolbenstange mit Handgriff 9a vorgesehen, die für ihre Benutzung teleskopartig verlängerbar beziehungsweise relativ zu dem Kolben 7 zunächst entgegen der Gebrauchsbewegung aus dem sie zunächst aufnehmenden Kolbenraum 2, also aus der in Fig. 1 und 2 sowie 7 dargestellten Position in die in den Fig. 3, 4 sowie 8 bis 14 dargestellte Lage ausziehbar beziehungsweise zurückziehbar ist. In dieser Gebrauchslage erfolgt gleichzeitig eine Kupplung dieser teleskopierbaren Kolbenstange 9 mit dem Kolben 7 mittels einer Verriegelung 10, die einerseits eine an dem Kolben 7 befindliche Zunge 11 und andererseits einen rampenartigen Vorsprung 12 an der Kolbenstange 9 umfasst.

Diese gegenseitige Verriegelung ist deutlich in den Fig. 8, 11, 12 und auch in Fig. 3 erkennbar. Wird nun die Kolbenstange 9 wieder in den Kolbenraum 2 hineingeschoben, wird der Kolben 7 mitgenommen, wie es Fig. 5 und 6 zeigt, so dass sein in Vorschubrichtung vor ihm angeordneter Stößel 8 das oder die Feststoffmedikamente 4 aus der Hohlnadel 5 ausschiebt.

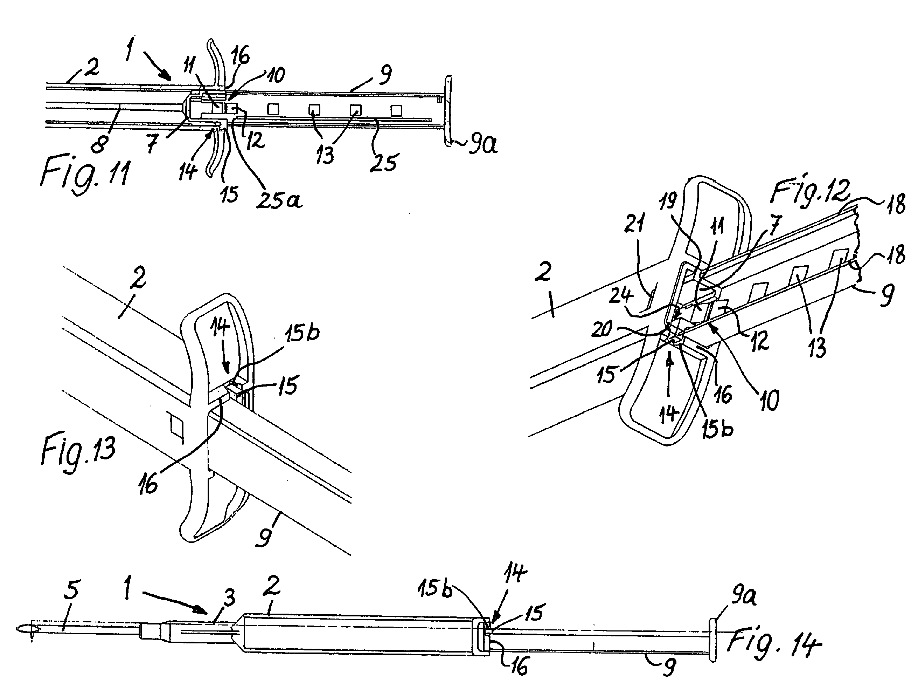

Vor allem in den Fig. 2 bis 5, 7, 8 und 12 erkennt man, dass die teleskopartig relativ zueinander verstellbaren Teile oder Bereiche des Kolbens 7 und vor allem der Kolbenstange 9 mehrere gegenseitige Sperren oder Verriegelungen für Zwischenpositionen der relativ zu dem Kolben 7 ausziehbaren Kolbenstange 9 aufweisen, welche Sperren bei nur teilweise relativ zu dem Kolben 7 zurückgezogener Kolbenstange 9 eine Verschiebung in Applikationsrichtung, also in den Zeichnungen von rechts nach links, verhindern. Vor allem anhand der Fig. 8 und 12 wird deutlich, dass außer dem zu der Verriegelung 10 gehörenden rampenartigen Vorsprung 12 an der inneren Unterseite der Kolbenstange 9 in deren Verlauf weitere rampenartige Vorsprünge 13 vorgesehen sind, die jeweils mit der Zunge 11 des Kolbens 7 zusammenwirken können, wenn eine nur teilweise ausgeführte Rückzugsbewegung der Kolbenstange 9 in eine Zwischenposition zwischen Fig. 2 und 3 zu einem Zusammenwirken der Zunge 11 mit einer diesen Rampen 13 im Sinne eines Gesperres führt.

Damit in einer solchen Zwischenposition das Zurückschieben der Kolbenstange 9 in das Kolbengehäuse 2 nicht zu einer Verschiebung des Kolbens 7 führt und das Medikament 4 nicht aufgrund der nur teilweisen Rückziehung auch nur teilweise ausgeschoben wird, ist eine insbesondere in den Fig. 11 bis 14 dargestellte, im Ganzen mit 14 bezeichnete Kupplung oder Verriegelung 14 des Kolbens 7 mit dem Kolbenraum 2 vorgesehen, die erst durch die vollständig in Gebrauchsstellung zurückgezogene Kolbenstange 9, in welcher die Kolbenstange 9 mit dem Kolben 7 durch die Verriegelung 10 in Schieberichtung verbunden ist, gelöst werden kann. Dadurch wird erreicht, dass also der Kolben 7 erst dann von dem Kolbengehäuse 2 in Schieberichtung gelöst wird, wenn die Kolbenstange 9 vollständig in die in den Fig. 3, 4 und 8 dargestellte Lage zurückgezogen ist, so dass auch der gesamte Verschiebeweg zum Applizieren des Medikaments 4 zur Verfügung steht. Fehlbedienungen durch eine nur um einen Teilbetrag zurückgezogene Kolbenstange 9 können also vermieden werden.

Vor allem in den Fig. 7, 8 und 11 bis 14 erkennt man, dass an dem Kolben 7 ein nachgiebiger oder in den Umriss des Kolbens 7 verstellbarer Vorsprung 15 vorgesehen ist, der zu der Kupplung oder Verriegelung 14 gehört und der bei in Ausgangslage befindlichem beziehungsweise zurückgezogenem Kolben 7 gemäß Fig. 2, 7 und 11 bis 14 an einer Kupplungsstelle des Kolbenraums 2 in eine Kupplungsvertiefung eingreift, die im Ausführungsbeispiel als randoffene Ausnehmung 15b an dem rückwärtigen Rand 16 des Kolbenraums 2 ausgebildet sein kann und das Einspringen und Einrasten dieses Vorsprungs 15 erlaubt, der ein Verschieben des Kolbens 7 in Applikationsrichtung verhindert. Dabei verdeutlichen die genannten Figuren, dass dieser an dem Kolben 7 angeordnete Vorsprung 15 eine Rast- oder Sperrklinke ist, die in Kupplungsstellung an einer sie unter- oder hintergreifenden Stelle des Kolbenraums 2 oder seines Gehäuses, nämlich der schon erwähnten Vertiefung oder Ausnehmung 15b, eingreift.

Dabei kann der Vorsprung 15 an seinem außenliegenden Randbereich seinerseits noch einen kleinen in Vorschubrichtung weisenden Vorsprung haben, der das Eindrücken dieser Sperrklinke mit dem Vorsprung 15 in das Innere des Kolbens erschwert, solange es nicht planmäßig durch die Kolbenstange 9 erfolgt, um Manipulationen und auch ungewollte Fehlbedienungen weitestgehend auszuschließen.

Wird also die Kolbenstange 9 nur soweit aus ihrer Ausgangslage gemäß Fig. 1 und 2 zurückgezogen, dass die zu der Verriegelung 10 gehörende Zunge 11 an einer der Rampen 13 einrastet, kann der Kolben 7 nicht in Applikationsrichtung verschoben werden, weil die zu der Verriegelung 14 gehörende Sperrklinke dies mit ihrem Vorsprung 15 durch dessen Verhakung mit dem Kolbenraum 2 in der Ausnehmung 15b verhindert.

Diese an dem Kolben 7 angeordnete Sperrklinke mit dem Vorsprung 15 ist durch eine mit der Sperrklinke verbundene schräge Führungsfläche 15a einerseits und einen an der Kolbenstange 9 angeordneten Vorsprungbereich 17 andererseits aus ihrer Kupplungs- oder Sperrstellung in den Umriss des Kolbens 7 gegen ihre Rückstellkraft soweit verschwenkbar oder verstellbar, dass diese Verriegelung 14 gelöst ist. Der genannte Vorsprungbereich 17 befindet sich dabei an der Kolbenstange 9 gemäß den Fig. 7 bis 10 nahe deren der Hohlnadel 5 zugewandten Ende, so dass also die Kolbenstange 9 tatsächlich bis zu der Position zurückgezogen werden muss, in welcher dann gleichzeitig die Verriegelung 10 stattfindet, um die zu der Kupplung oder Verriegelung 14 gehörende Sperrklinke außer Funktion zu bringen.

Dabei ist in den Fig. 2, 4, 7 und 11 noch eine Begrenzungsrippe 25 angedeutet, die in Schieberichtung neben den Rampen 13 innerhalb der Kolbenstange 9 verläuft und mit etwas Abstand vor dem in Vorschubrichtung vorderen Ende der Kolbenstange 9 endet und die ein Verschwenken der den Vorsprung 15 aufweisenden Sperrklinke in das Innere des Kolbens 7 solange verhindert, bis dieses vordere Ende 25a dieser Begrenzungsrippe 25 den Kolbenbereich verlassen hat, was in Fig. 4 und 11 dargestellt ist. Erst in dieser vollständig zurückgezogenen Lage kann also der Vorsprung 15 nach innen verschwenkt werden, während dies bei einer Zwischenstellung der Kolbenstange durch die Begrenzungsrippe 25 verhindert ist, so dass in einer Zwischenstellung auch keine Manipulation an der Kolbenverriegelung möglich ist.

Der Benutzer zieht also die Kolbenstange 9 aus der in Fig. 2 und Fig. 7 dargestellte Lage in die in den Fig. 3, 4 und 8 bis 14 gezeigte Position zurück, wodurch einerseits die in Rückzugsrichtung letzte Rampe 12 mit der Zunge 11 der Verriegelung 10 in Gebrauchskontakt und in Funktionsstellung gelangt, während gleichzeitig die Verriegelung 14 durch ein Einschwenken des Vorsprunges 15 an der Sperrklinke in den Umriss des Kolbens 7 außer Funktion gelangt. Fig. 8 und 11 bis 14 zeigen dabei der größeren Deutlichkeit wegen den Vorsprung 15 noch kurz vor seiner Zurückverschwenkung in das Innere des Kolbens 7, obwohl die Kolbenstange 9 schon soweit zurückgezogen ist, dass sie selbst mit dem Kolben 7 gekuppelt ist. In Wirklichkeit ist in dieser Kupplungsposition, in welcher die Kolbenstange 9 und der Kolben 7 für eine gemeinsame Schiebebewegung in Richtung zu der Kanüle 5 und dem Medikament 4 hin formschlüssig verbunden sind, die Verriegelung 14 in der schon beschriebenen Weise gelöst.

Dies alles ist möglich, weil der Kolben 7 analog der in EP 1 323 450 B1 beschriebenen Vorrichtung innerhalb der ein Hohlprofil bildendes beziehungsweise rinnenförmiges Querschnittsprofil aufweisenden Kolbenstange 9 angeordnet ist und diese den Kolben 7 und wenigstens einen Teil seines Stößels 8 vor der Zurückziehung der Kolbenstange 9 in die Gebrauchsstellung platzsparend in sich aufnimmt, was vor allem anhand der Fig. 1, 2 und 7 deutlich wird. Der den Kolben 7 aufnehmende Teil der Kolbenstange 9 ist also zwischen Kolben 7 und Kolbenraum 2 relativ zu diesen beiden in die Gebrauchsstellung zurückziehbar.

Durch die zu der Sperre 10 gehörende Zunge 11 des Kolbens 7 und die rampenartigen schrägen Sperrvorsprünge 13 der Kolbenstange 9 weisen also die relativ zueinander in Längsrichtung teleskopartig verstellbaren Teile, nämlich die Kolbenstange 9 und der relativ zu ihr verschiebbare Kolben 7, der bei der Benutzung allerdings unverstellt bleibt, weil die Kolbenstange 9 die Verschiebebewegung ausführt, als gegenseitige Sperren in mehreren Zwischenstellungen Schnappverbindungen auf, die, wie schon beschrieben, eine Kupplung zwischen Kolbenstange 9 und Kolben 7 in Betätigungsrichtung des Kolbens 7 bewirken, welche Betätigung des Kolbens 7 aber durch die Kupplung des Kolbens 7 mit den Kolbenraum 2 mittels der den Vorsprung 15 aufweisenden Sperrklinke in solchen Zwischenstellungen gesperrt bleibt. Die Schnappverbindungen sind durch die an der Innenseite der Kolbenstange 9 angeordneten Rampen oder Zungen 13 gebildet, die ihrerseits mit der federelastischen Zunge 11 an dem Kolben 7 zusammenwirken, welche Zunge 11 durch die Rampen 13 beim Zurückziehen der Kolbenstange 9 zunächst eindrückbar ist und dann jeweils hinter eine solche Rampe 13 hörbar in Sperrstellung bewegbar ist und einschnappt. Dabei wird vor allem aus Fig. 8 und 12 deutlich, dass die an dem Kolben 7 befindliche Zunge 11 an einer Kolbenbegrenzung angeordnet ist, die etwa rechtwinklig zu jener Kolbenbegrenzung verläuft, an welcher der Vorsprung 15 für die Sperrklinke vorsteht.

Beim Zurückziehen der Kolbenstange aus der Ausgangslage in die Gebrauchsstellung ertönen also bei jeder Berührung zwischen Zunge 11 und einer der Rampen 13 Klickgeräusche, die den Benutzer darauf aufmerksam machen können, dass entsprechende Zwischenstellungen erreicht sind. Nach einem letzten Klickgeräusch, nach welchem die Kolbenstange 9 nicht weiter aus dem Kolbenraum 2 herausziehbar ist, ist dann gleichzeitig der Kolben 7 gegenüber dem Kolbenraum 2 durch Zurückverschwenken der Sperrklinke mit dem Vorsprung 15 in das Innere des Umrisses des Kolbenraumes 2 entriegelt, so dass der Applikationsvorgang über die gesamte Schublänge der Kolbenstange 9 durchgeführt werden kann.

Aus den Zeichnungen und vor allem Fig. 8 und 12 ergibt sich, dass im Ausführungsbeispiel die Kolbenstange 9 als rinnenartiges Teil ausgebildet ist, dessen Innenquerschnitt u-förmig ist und den Kolben 7 über einen Teil von dessen Querschnitt oder Umriss umfasst und führt. Dabei übergreift der Kolben 7 die freien Ränder 18 dieses U-Querschnitts der Kolbenstange 9 mittels Vorsprüngen 19 und 20 seitlich um den Betrag, der etwa der Stärke oder Dicke der Wandungen der Kolbenstange 9 entspricht, so dass der Kolben 7 auch außerhalb der Kolbenstange 9 in den Kolbenraum 2 passt und von dessen Innenquerschnitt geführt wird, wie dies auch für die Kolbenstange 9 und deren Außenumriss zutrifft. Praktisch ergänzt der Kolben 7 mit seinem Außenquerschnitt den Außenquerschnitt der Kolbenstange 9 so, dass er etwa dem Innenquerschnitt des Kolbenraums 2 entspricht und die Führung darin in Verschieberichtung erlaubt.

Damit die in Fig. 7 dargestellte Einheit aus Kolbenstange 9 und Kolben 7 in zwei um 180° gewendeten Positionen in den Kolbenraum 2 passt, ist für die Verriegelung des Kolbens in der Endstellung nach der Applikation mit Hilfe einer Sperrzunge 21 an dem Kolbenraum 2 an zwei gegenüberliegenden Stellen eine Ausnehmung 22 vorgesehen, der auch eine entsprechende Ausnehmung 23 für die Ausgangslage des Kolbens entspricht, um diese Zunge 21 - in Applikationsrichtung verschiebbar - aufzunehmen.

Damit die den Vorsprung 15 und die Führungsfläche 17 aufweisende Sperrklinke sich trotz des oberhalb von ihr angeordneten Vorsprungs 20 gut bewegen lässt, im Bereich dieser Sperrklinke aber auch die innenseitige Führung des Kolbens 7 mit seinem Vorsprung 20 problemlos möglich ist, ist die die Sperrklinke aufweisende Seite des Kolbens 7 zum Abgrenzen dieser Sperrklinke von der Kolbenseitenfläche geschlitzt und man erkennt diesen Schlitz 24 unter dem Vorsprung 20 an der Seite des Kolbens in den Fig. 9 und 10. Die außerhalb oder oberhalb der Kolbenstange 9 befindliche Begrenzung dieses Schlitzes 24 steht dabei als Vorsprung 20 seitlich etwa um die Dicke der Seitenwand der Kolbenstange 9 über, so dass der Kolben 7 an seinem außerhalb der teleskopierbaren Kolbenstange 9 beziehungsweise außerhalb von deren Querschnitt befindlichen Bereich zusammen mit dem auf der anderen Seite angeordneten Vorsprung 19 eine Gesamtbreite hat, die der Gesamtbreite der Kolbenstange 9 und der inneren Breite des Kolbenraumes 2 entspricht, was zu einer guten Führung der Gesamtanordnung aus Kolben 7 und Kolbenstange 9 beiträgt.

Die Spritzenvorrichtung 1 weist einen länglichen Kolbenraum 2, eine Aufnahmekammer 3 zum Aufnehmen von Feststoffmedikamenten 4 und eine an dem Kolbenraum 2 angeordnete Hohlnadel 5 sowie einen zu einer Bewegung im Kolbenraum 2 geeigneten Kolben 7 auf, der einen Stößel 8 zum Ausschieben des Feststoffmedikaments 4 trägt. In Ausgangslage ist der Kolben 7 dem Feststoffmedikament 4 möglichst weit abgelegen mittels einer Verriegelung in dem Kolbenraum 2 zunächst unverschiebbar gehalten. Für die Verschiebung des Kolben 7 ist eine an ihm angreifende Kolbenstange 9 vorgesehen, die für ihre Benutzung teleskopartig aus dem Kolbenraum 2 und relativ zu dem Kolben 7 ausziehbar und mittels einer Verriegelung 10 mit dem Kolben kuppelbar ist. Die Verriegelung des Kolbens 7 mit dem Kolbenraum 2 wird nur durch eine vollständige Zurückziehung der Kolbenstange 9 in deren Gebrauchsstellung geöffnet oder entriegelt, während gleichzeitig die Kolbenstange 9 mit dem Kolben 7 in dieser Lage in Schieberichtung gekuppelt wird, so dass dann das Medikament ausgeschoben werden kann. Damit der Benutzer keine Fehlbedienung durch teilweises Zurückziehen der Kolbenstange 9 durchführen kann, weisen der Kolben 7 und die Kolbenstange 9 wenigstens eine oder mehrere gegenseitige Sperren für Zwischenpositionen auf, in denen aber der Kolben 7 noch mit dem Kolbenraum 2 verriegelt ist.

## Patentansprüche

1. Spritzenvorrichtung (1) mit einem länglichen Kolbenraum (2) oder Zylinder, mit einer Aufnahmekammer (3) zum Aufnehmen einer oder mehrerer Feststoffmedikamente (4), mit einer an dem Kolbenraum (2) angeordneten Hohlnadel (5) und mit einem zu einer Bewegung in dem Kolbenraum (2) oder Zylinder geeigneten Kolben (7), der einen Stößel (8) zum Ausschieben des Feststoffmedikaments (4) durch und aus der Hohlnadel (5) aufweist, wobei eine an dem Kolben (7) angreifende Kolbenstange (9) für ihre Benutzung teleskopartig verlängerbar beziehungsweise relativ zu dem Kolbenraum (2) und zu dem Kolben (7) entgegen dessen Gebrauchsbewegung ausziehbar und mittels einer Verriegelung (10) mit dem Kolben (7) kuppelbar ist, **dadurch gekennzeichnet, dass** die teleskopartig relativ zueinander verstellbaren Teile oder Bereiche des Kolbens (7) und der Kolbenstange (9) wenigstens eine oder mehrere gegenseitige Sperren für Zwischenpositionen der relativ zu dem Kolben (7) ausziehbaren Kolbenstange (9) aufweisen, welche Sperre oder Sperren bei nur teilweise relativ zu dem Kolben (7) zurückgezogener Kolbenstange (9) eine Verschiebung in Applikationsrichtung verhindern, wobei eine Kupplung oder Verriegelung (14) des Kolbens (7) in seiner Ausgangslage vorgesehen ist, die durch die in Gebrauchsstellung zurückgezogene Kolbenstange (9), in welcher die Kolbenstange (9) mit dem Kolben (7) in Schieberichtung verbunden ist, lösbar ist.

2. Spritzenvorrichtung nach Anspruch 1, **dadurch gekennzeichnet, dass** an dem Kolben (7) ein nachgiebiger oder in den Umriss des Kolbens (7) verstellbarer Vorsprung (15) vorgesehen ist, der bei zurückgezogenem Kolben (7) an einer Kupplungsstelle des Kolbenraums (2) selbsttätig in eine Kupplungsvertiefung oder Ausnehmung (15b) eingreift.

3. Spritzenvorrichtung nach Anspruch 2, **dadurch gekennzeichnet, dass** der an dem Kolben (7) angeordnete Vorsprung (15) eine Rast- oder Sperrklinke ist, die in Kupplungsstellung an einer sie untergreifenden Stelle des Kolbenraums (2) oder seines Gehäuses eingreift.

4. Spritzenvorrichtung nach Anspruch 3, **dadurch gekennzeichnet, dass** die an dem Kolben (7) angeordnete Sperrklinke durch eine mit der Sperrklinke verbundene Führungsfläche (15a) und einen an der Kolbenstange (9) angeordneten Vorsprungbereich (17) aus ihrer Kupplungs- oder Sperrstellung in den Umriss des Kolbens (7) gegen ihre Rückstellkraft verstellbar ist, welcher Vorsprungbereich (17) an der Kolbenstange (9) nahe deren der Hohlnadel (5) zugewandten Ende angeordnet ist.

5. Spritzenvorrichtung nach einem der Ansprüche 1 bis 4, **dadurch gekennzeichnet, dass** der Kolben (7) innerhalb der ein Hohlprofil bildendes oder rinnenförmiges Querschnittsprofil aufweisenden Kolbenstange (9) angeordnet und diese den Kolben (7) und wenigstens einen Teil seines Stößels (8) vor der Gebrauchsstellung in sich aufnimmt.

6. Spritzenvorrichtung nach einem der Ansprüche 1 bis 5, **dadurch gekennzeichnet, dass** die relativ zueinander in Längsrichtung teleskopartig verstellbaren Teile, nämlich der Kolben (7) und die relativ zu ihm verschiebbare Kolbenstange (9) als gegenseitige Sperren in mehreren Zwischenstellungen Schnappverbindungen aufweisen, die eine Kupplung zwischen Kolbenstange (9) und Kolben (7) in Betätigungsrichtung des Kolbens (7) bewirken, welche Betätigung des Kolbens (7) durch die Kupplung des Kolbens (7) mit dem Kolbenraum (2) mittels Sperrklinke in den Zwischenstellungen gesperrt ist.

7. Spritzenvorrichtung nach Anspruche 6, **dadurch gekennzeichnet, dass** die Schnappverbindungen durch an der Innenseite der Kolbenstange (9) angeordnete Rampen oder Zungen (13) gebildet sind, die ihrerseits mit einer insbesondere federelastischen Zunge (11) an dem Kolben (7) zusammenwirken, welche Zunge (11) durch die Rampen (13) beim Zurückziehen der Kolbenstange (9) zunächst eindrückbar ist und jeweils hinter der Rampe (13) durch ihre Rückstellkraft insbesondere hörbar in ihre Sperrstellung bewegbar ist.

8. Spritzenvorrichtung nach einem der Ansprüche 1 bis 7, **dadurch gekennzeichnet, dass** die Kolbenstange (9) als rinnenartiges Teil ausgebildet ist, in dessen Innenquerschnitt der Kolben (7) über einen Teil seines Querschnitts umfasst und geführt ist, und dass der Kolben (7) die freien Ränder (18) des U-Querschnitts der Kolbenstange (9) mittels Vorsprüngen (19, 20) seitlich übergreift, die zusammen mit der Kolbenstange in den Kolbenraum (2) passen und von dessen Innenquerschnitt geführt sind.

9. Spritzenvorrichtung nach einem der Ansprüche 3 bis 8, **dadurch gekennzeichnet, dass** die die Sperrklinke aufweisende Seite des Kolbens (7) zum Abgrenzen der Sperrklinke von der Kolbenseitenfläche geschlitzt ist und die außerhalb der Kolbenstange (9) befindliche Begrenzung dieses Schlitzes (24) über den Kolben seitlich etwa um die Dicke der Seitenwand der Kolbenstange (9) übersteht, so dass der Kolben an seinem außerhalb der teleskopierbaren Kolbenstange (9) befindlichen Bereich eine Gesamtbreite hat, die der Gesamtbreite der Kolbenstange (9) und der inneren Breite des Kolbenraumes (2) entspricht.

## Claims

1. Injection apparatus (1) having an elongate piston chamber (2) or cylinder, having a receiving chamber (3) for holding one or more solid medicaments (4), having a hollow needle (5) arranged on the piston chamber (2) and having a piston (7) that is suitable for moving in the piston chamber (2) or cylinder, which comprises a ram (8) for pushing the solid medicament (4) through and out of the hollow needle (5), while a piston rod (9) acting on the piston (7) can be extended telescopically for use or pulled out relative to the piston chamber (2) and to the piston (7), counter to the movement of use thereof, and can be coupled to the piston (7) by means of a latch (10), **characterised in that** the parts or regions of the piston (7) and of the piston rod (9) that can be moved telescopically relative to one another have at least one or more mutual barriers for intermediate positions of the piston rod (9) that can be extended relative to the piston (7), the said barrier or barriers preventing movement in the direction of use if the piston rod (9) is only partly withdrawn relative to the piston (7), while means (14) for coupling or latching the piston (7) in its starting position are provided, which can be undone by the piston rod (9) pulled back into the position of use in which the piston rod (9) is connected to the piston (7) in the direction of pushing.

2. Injection apparatus according to claim 1, **characterised in that**, provided on the piston (7) is a projection (15) which is resilient or movable into the contour of the piston (7), which when the piston (7) is pulled back at a coupling point of the piston chamber (2) engages automatical in a coupling recess or depression (15b),

3. Injection apparatus according to claim 2, **characterised in that** the projection (15) provided on the piston (7) is a latching or locking pawl which in the coupling position acts on a point of the piston chamber (2) or its housing that engages underneath it.

4. Injection apparatus according to claim 3, **characterised in that** the locking pawl arranged on the piston (7) can be moved, counter to its restoring force, by a guide surface (16a) connected to the locking pawl and by a projecting region (17) arranged on the piston rod (9), out of its coupling or locking position into the contour of the piston (7), this projecting region (17) being arranged on the piston rod (9) close to its end facing the hollow needle (5).

5. Injection apparatus according to one of claims 1 to 4, **characterised in that** the piston (7) is arranged within the piston rod (9) that forms a hollow profile or a channel-shaped cross-sectional profile, and the piston rod (9) receives the piston (7) and at least part of its ram (8) inside it before the position of use.

6. Injection apparatus according to one of claims 1 to 5, **characterised in that** the parts that are telescopically movable relative to one another in the longitudinal direction, namely the piston (7) and the piston rod (9) that is movable relative thereto, comprise, as mutual barriers in a number of intermediate positions, snap-fit confections which bring about coupling between the piston rod (9) and the piston (7) in the direction of actuation of the piston (7), this actuation of the piston (7) being blodked by the coupling of the piston (7) to the piston chamber (2) by a locking pawl in the intermediate positions.

7. Injection apparatus according to claim 6, **characterised in that** the snap-fit confections are formed by ramps or tongues (13) provided on the inside of the piston rod (9), which in tum cooperate with an in particular springily elastic tongue (11) on the piston (7), this tongue (11) being initially capable of being pressed in by the ramps (13) as the piston rod (9) is pulled back and being in particular audibly movable by its restoring force into its locking position behind the ramp (13) in each case.

8. Injection apparatus according to one of claims 1 to 7, **characterised in that** the piston rod (9) is constructed as a channel-like part within whose internal cross-section the piston (7) is encompassed and guided, over part of its cross-section, and **in that** the piston (7) laterally overlaps the free edges (18) of the U-shaped cross-section of the piston rod (9) by means of projections (19, 20) which together with the piston rod fit into the piston chamber (2) and are guided by the internal cross-section thereof.

9. Injection apparatus according to one of claims 3 to 8, **characterised in that** the side of the piston (7) that comprises the locking pawl is slotted to distinguish the locking pawl from the lateral piston surface and the boundary of this slot (24) located outside the piston rod (9) projects laterally somewhat over the piston by the thickness of the side wall of the piston rod (9), so that the piston has an overall width, at its region located outside the telescoping piston rod (9), which corresponds to the total width of the piston rod (9) and the internal width of the piston chamber (2).

## Revendications

1. Dispositif d'injection (1), avec une chambre de piston oblongue (2) ou un cylindre, avec une chambre de réception (3) pour recevoir un ou plusieurs médicaments solides (4), avec une aiguille creuse (5) disposée sur la chambre de piston (2), et avec un piston (7) qui peut se déplacer dans la chambre de piston (2) ou le cylindre et qui présente un poussoir (8) pour expulser le médicament solide (4) à travers et hors de l'aiguille creuse (5), sachant qu'une tige de piston (9) agissant sur le piston (7) peut, pour être utilisée, être allongée télescopiquement ou encore déployée par rapport à la chambre de piston (2) et au piston (7) à l'encontre du mouvement d'utilisation de ce dernier, et accouplée au piston (7) au moyen d'un verrouillage (10), **caractérisé en ce que** les éléments ou les régions du piston (7) et de la tige de piston (9) qui sont mobiles télescopiquement l'un par rapport à l'autre présentent au moins un ou plusieurs blocages mutuels pour des positions intermédiaires de la tige de piston (9) pouvant être déployée par rapport au piston (7), ce ou ces blocages empêchant un déplacement dans la direction d'application lorsque la tige de piston (9) n'est que partiellement reculée par rapport au piston (7), sachant qu'il est prévu un accouplement ou verrouillage (14) du piston (7) dans sa position initiale, qui peut être libéré par la tige de piston (9) reculée dans la position d'utilisation, dans laquelle la tige de piston (9) est reliée au piston (7) dans la direction de poussée.

2. Dispositif d'injection selon la revendication 1, **caractérisé en ce qu'**il est prévue sur le piston (7) une saillie (15) flexible ou déplaçable dans le contour du piston (7), laquelle, lorsque le piston (7) est reculé, s'engage automatiquement dans un renfoncement d'accouplement ou évidement (15b) en un point d'accouplement de la chambre de piston (2).

3. Dispositif d'injection selon la revendication 2, **caractérisé en ce que** la saillie (15) disposée sur le piston (7) est un cliquet d'arrêt ou de blocage qui, dans la position d'accouplement, s'engage en un point de la chambre de piston (2) ou de son enveloppe qui vient en prise sous lui.

4. Dispositif d'injection selon la revendication 3, **caractérisé en ce que** le cliquet de blocage disposé sur le piston (7) peut être déplacé hors de sa position d'accouplement ou de blocage dans le contour du piston (7), à l'encontre de sa force de rappel, par une surface de guidage (15a) reliée au cliquet d'arrêt et une région en saillie (17) disposée sur la tige de piston (9), la région en saillie (17) étant disposée sur la tige de piston (9) à proximité de l'extrémité de celle-ci qui est tournée vers l'aiguille creuse (5).

5. Dispositif d'injection selon l'une des revendications 1 à 4, **caractérisé en ce que** le piston (7) est disposé à l'intérieur de la tige de piston (9) formant un profilé creux ou présentant un profil de section en forme de goulotte, et celle-ci reçoit à l'intérieur d'elle le piston (7) et au moins une partie de son poussoir (8) avant d'atteindre la position d'utilisation.

6. Dispositif d'injection selon l'une des revendications 1 à 5, **caractérisé en ce que** les éléments mobiles télescopiquement l'un par rapport à l'autre en direction longitudinale, à savoir le piston (7) et la tige de piston (9) pouvant coulisser par rapport à ce dernier, présentent comme blocages mutuels dans plusieurs positions intermédiaires des liaisons par enclenchement qui produisent un accouplement entre la tige de piston (9) et le piston (7) dans la direction d'actionnement du piston (7), cet actionnement du piston (7) étant bloqué dans les positions intermédiaires par l'accouplement du piston (7) avec la chambre de piston (2) au moyen du cliquet de blocage.

7. Dispositif d'injection selon la revendication 6, **caractérisé en ce que** les liaisons par enclenchement sont formées par des rampes ou languettes (13) disposées sur le côté intérieur de la tige de piston (9), qui coopèrent elles-mêmes avec une languette (11) notamment à élasticité de ressort sur le piston (7), la languette (11) pouvant être d'abord enfoncée par les rampes (13) lors du recul de la tige de piston (9), et respectivement déplacée dans sa position de blocage en arrière de la rampe (13) par sa force de rappel, notamment de manière audible.

8. Dispositif d'injection selon l'une des revendications 1 à 7, **caractérisé en ce que** la tige de piston (9) est réalisée sous la forme d'un élément du genre goulotte, dans la section intérieure de laquelle le piston (7) est contenu et guidé sur une partie de sa section, et **en ce que** le piston (7) engage en recouvrement latéral les bords libres (18) de la section en U de la tige de piston (9) au moyen de saillies (19, 20) qui s'ajustent avec la tige de piston dans la chambre de piston (2) et sont guidées par la section intérieure de celle-ci.

9. Dispositif d'injection selon l'une des revendications 3 à 8, **caractérisé en ce que** le côté du piston (7) qui présente le cliquet de blocage est fendu afin de délimiter le cliquet de blocage par rapport à la face latérale du piston, et la délimitation de cette fente (24) qui se trouve en dehors de la tige de piston (9) s'étend latéralement au-delà du piston environ de l'épaisseur de la paroi latérale de la tige de piston (9), de sorte que le piston possède, dans sa région se trouvant en dehors de la tige de piston télescopique (9), une largeur totale qui correspond à la largeur totale de la tige de piston (9) et à la largeur intérieure de la chambre de piston (2).
